# EUROPÄISCHE PATENTSCHRIFT

(11) **EP 1 642 552 B1**
(45) Veröffentlichungstag und Bekanntmachung des Hinweises auf die Patenterteilung: **12.11.2008**
(21) Anmeldenummer: 05017885.4
(22) Anmeldetag: 17.08.2005
(51) Int. Cl.: A61F 2/42

(54) **Endoprothese für ein Metatarsophalangealgelenk**
Endoprothesis for a metatarsophalangeal joint
Endoprothèse pour une articulation metatarsophalangienne

(30) Priorität: 09.09.2004 DE 102004043700
(43) Veröffentlichungstag der Anmeldung: 05.04.2006
(73) Patentinhaber: Plus Orthopedics AG, 6343 Rotkreuz (CH)
(72) Erfinder: Frey, Theo, 8932 Mettmenstetten (CH)
(74) Vertreter: Popp, Eugen

(56) Entgegenhaltungen:
- EP-B- 0 827 386
- DE-A1- 10 134 511
- US-A- 4 642 122
- US-B1- 6 319 284

## Beschreibung

Die Erfindung betrifft eine Endoprothese für ein Metatarsophalangeal (MTP)-Gelenk, insbesondere Großzehengrundgelenk, Zehen- oder Fingergelenk, mit einer eine konkave oder konvexe Ersatzgleitfläche definierenden Prothesenhälfte.

Eine derartige Prothese ist z.B. bekannt aus der EP 0 827 386 B1, die auf die Anmelderin zurückgeht. Ergänzend dazu sei darauf hingewiesen, daß der Gelenkersatz für ein Zehengrundgelenk, insbesondere für eine Großzehe heute darin besteht, entweder die Gelenkfläche distal (Grundphalanx) mit einer Prothesenhälfte bzw. Hemiprothese, oder distal und proximal (Grundphalanx und Metatarsale) mit einer Totalprothese zu ersetzen. Dabei kommen sowohl monoblocke als auch modulare Endoprothesen zum Einsatz. Dieser künstliche Gelenkersatz ist meist aus Metall, Kobaltchrom- oder Titanleglierungen oder Reintitan gefertigt, wobei auch Keramik oder Polyethylen verwendet wird. Modulare Endoprothesen für die Großzehe werden vorwiegend zementfrei mit sogenanntem "Press-fit" oder mittels Schraubverankerung im Knochen verankert.

Die proximale Komponente von Totalprothesen hat einen axialzentrierten oder gekröpften Gelenkflächenersatz in Form eines Kugelsegments.

Die distale Komponente weist eine zentriert zur Achse des Verankerungszapfens oder Stiels angeordnete Scheibe mit Vertiefung bzw. Pfanne auf, welche als Gleitlager für die proximale Komponente wirkt.

Es hat sich nun gezeigt, daß es intraoperativ sehr schwierig ist, das Implantat im Knochen zu zentrieren. Es kann ein Überhang des Gelenkeinsatzes entstehen, welcher zu Irritationen an den Weichteilen oder der Gelenkkapsel führt. Andererseits kann dieser Überhang auch eine Reduktion des Bewegungsumfangs herbeiführen, da es durch die Erhöhung des Implantates zu höherer Weichteilspannung kommen kann. In extremen Fällen kommt es zu Luxationen.

Die US 4,642,122 beschreibt eine Endoprothese für ein Metatarsophalangealgelenk mit einer eine konkave bzw. konvexe Ersatzgleitfläche definierenden Prothesenhälfte mit exzentrisch zu den Ersatzgleitflächen der Prothesenhälfte angeordneten Mitteln zur Verankerung derselben im Knochen, wobei diese Mittel als Verankerungszapfen ausgebildet sein können.

Die in der US 4,542,122 beschriebene Prothesenhälfte ist hinsichtlich der dortigen Ersatzgleitfläche und der dortigen Mittel zur Verankerung im Knochen einstückig ausgebildet, so daß hinsichtlich der Ersatzgleitfläche und der jeweiligen Verankerungszapfen keine Variabilität besteht, da diese untrennbar miteinander verbunden sind. Somit weist die dortige Endoprothese den Nachteil auf, daß je nach Größe der zu versorgenden Knochen eine exakt an deren Größe angepaßte Endoprothese verwendet werden muß, da die Zuordnung unterschiedlicher Ersatzgleitflächen zu ein und dem selben Verankerungszapfen aufgrund der dortigen einstückigen Ausbildung dieser beiden Teile nicht möglich ist.

Somit besteht die Aufgabe der Erfindung darin, eine Endoprothese zur Verfügung zu stellen, die in Weiterbildung der in der US 4,642,122 dargelegten Vorrichtung eine erhöhte, insbesondere operative, Variabilität bei einer gleichzeitig kostengünstigen Herstellung der Endoprothese ermöglicht. Ferner liegt der vorliegenden Erfindung die Aufgabe zugrunde, eine Endoprothese zur Verfügung zu stellen, mit der der erwähnte Überhang speziell im dorsalen oder plantaren Bereich des Metatarsal- oder Phalangealknochenendes vermieden werden kann. Insbesondere soll durch eine zentrierte Ausrichtung von Metatarsale auf Grundphalanx oder umgekehrt die Gefahr einer Luxation reduziert werden können. Dabei soll die erfindungsgemäße Endoprothese so ausgebildet sein, daß auf aufwendige Ziel-Führungsinstrumente verzichtet werden kann. Insbesondere soll es unschädlich sein, wenn die Implantatteile unzentriert implantiert werden; denn es soll möglich sein, anschließend die Implantatteile zu justieren und an den jeweiligen Patienten anzupassen.

All diese Teilaufgaben werden erfindungsgemäß in äußerst einfacher und eleganter Weise durch die kennzeichnenden Merkmale des Anspruches 1 gelöst, wobei bevorzugte Details und Weiterentwicklungen sowie Ausführungsformen in den Unteransprüchen beschrieben sind.

Für die Erfindung ist also u.a. von Bedeutung, daß die Mittel zur Verankerung der Prothesenhälfte im Knochen derart ausgebildet sind, daß die konkave bzw. konvexe Ersatzgleitfläche relativ dazu, d.h. zu den Verankerungsmitteln, entweder in eine zentrische oder bei Bedarf exzentrische Lage bringbar ist. Auf diese Weise ist es auch bei unzentrierter Implantation einer Prothesenhälfte möglich, die zugeordnete Ersatzgleitfläche nachzujustieren und an den jeweiligen Patienten zu adaptieren, ohne daß besondere Instrumente dafür erforderlich wären. Die erfindungsgemäß vorgesehene Exzentrizität erlaubt eine Nachjustierung einer Prothesenhälfte durch einfaches Drehen, bis die gewünschte Position erreicht ist und bevor die endgültige Verkoppelung der Prothesenhälfte im Knochen oder an einem vorher im Knochen zementfrei oder durch Zementierung verankertes Anschlußelement erfolgt. Die Justierung der Prothesenhälfte bzw. der konkaven oder konvexen Ersatzgleitfläche derselben erfolgt also intraoperativ durch den Chirurgen.

Die Prothesenhälften sind modular ausgebildet.

Die Erfindung ist ferner dadurch gekennzeichnet, daß zur Verankerung der Prothesenhälften diese jeweils einen Verankerungszapfen aufweisen, dessen Mittenachse relativ zur Mittenachse der konkaven bzw. konvexen Ersatzgleitfläche versetzt ist. Vorzugsweise ist der Verankerungszapfen konisch ausgebildet und somit selbsthemmend verankerbar, vorzugsweise innerhalb einer komplementär konischen Aufnahmebohrung eines im Knochen verankerbaren Anschlußelements.

Dabei ist es auch denkbar, daß gegebenenfalls auch die Zapfenaufnahme exzentrisch angeordnet ist, vorzugsweise mit derselben Exzentrizität wie der Verankerungszapfen an der Prothesenhälfte, so daß die Prothesenhälfte in eine zentrierte Position bringbar ist.

Die Mittenachsen von Ersatzgleitfläche und Prothesenverankerung erstrecken sich in der Regel parallel zueinander. Für bestimmte Korrekturzwecke ist es jedoch auch denkbar, daß die erwähnten Mittenachsen sich in einem vorbestimmten, insbesondere spitzen Winkel zueinander erstrecken. Damit ist es möglich, z.B. die Großzehenstellung zu korrigieren, ohne daß es einer aufwendigen intrakapitalen Keilosteotomie oder basalen Keilosteotomie bedarf. Die Ansprüche 7 und 8 betreffen bevorzugte Schraubfixierungen der Prothesenhälfte an einem im Knochen verankerbaren Anschlußelement. Die erste Variante ist dadurch gekennzeichnet, daß die Prothesenhälfte oder ein Befestigungsteil derselben einen geradlinig oder bogenförmig verlaufenden langlochartigen Durchgang für eine Befestigungsschraube aufweist, der im Anschlußelement eine Gewindebohrung zugeordnet ist. Vorzugsweise liegt der erwähnte Durchgang außerhalb der orthopädisch wirksamen Ersatzgleitflächen.

Die alternative Ausführungsform einer Schraubfixierung umfaßt ein Anschlußelement mit mehreren voneinander beabstandeten Gewindebohrungen zur Aufnahme einer der Prothesenhälfte zugeordneten Befestigungsschraube, wobei die Gewindebohrungen so angeordnet sind, daß die Prothesenhälfte mit ihrer Ersatzgleitfläche entweder zentrisch oder bei Bedarf exzentrisch zur Mittenachse des Anschlußelements an diesem befestigbar ist.

Alternativ zu dieser letztgenannten Lösung ist es auch denkbar, in der Prothesenhälfte ein im Vergleich zum Durchmesser der Befestigungsschraube etwas größeres Durchgangsloch oder ein etwas breiteres Langloch auszubilden, um die Prothesenhälfte vor der endgültigen Blockierung mittels der Befestigungsschraube nach allen Richtungen hin verschieben zu können, und zwar entweder in eine zentrierte oder exzentrische Lage. Letztgenannte Alternative ist in Anspruch 9 festgehalten.

Nachstehend werden bevorzugte Ausführungsformen der erfindungsgemäßen Endoprothese anhand der beigefügten Zeichnung näher beschrieben. Diese zeigt in:
- Fig. 1: eine proximale Prothesenhälfte in Seitenansicht;
- Fig. 2: die proximale Prothesenhälfte gemäß Fig. 1 in Ansicht von hinten bzw. proximal;
- Fig. 3: eine distale Prothesenhälfte in Seitenansicht;
- Fig. 4: die distale Prothesenhälfte gemäß Fig. 3 in Ansicht von hinten bzw. von distal;
- Fig. 5: eine Totalprothese mit exzentrisch positionierter proximaler Prothesenhälfte in Seitenansicht; und
- Fig. 6: eine Hemiprothese mit exzentrisch positionierter distaler Prothesenhälfte in Seitenansicht.

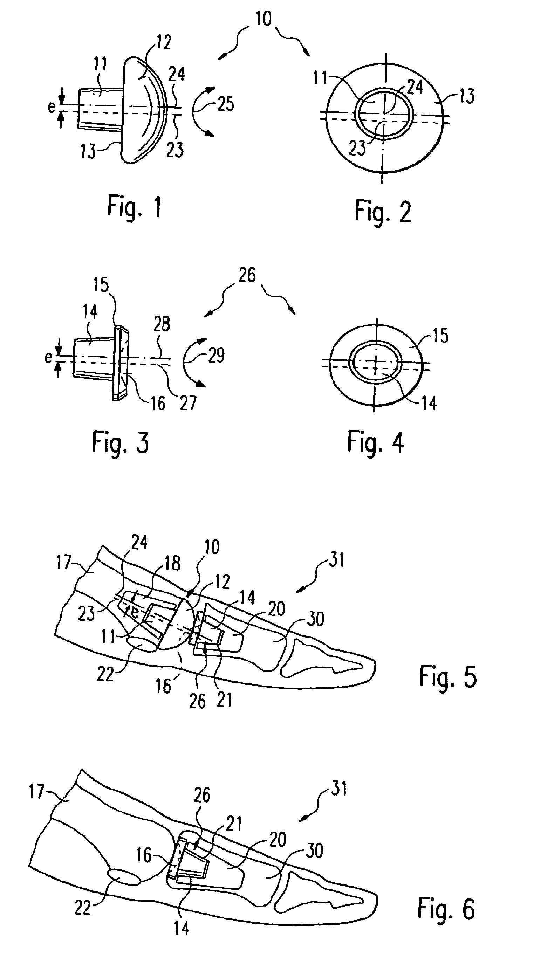

Der proximale Gelenkeinsatz bzw. die proximale Prothesenhälfte gemäß den Figuren 1 und 2 umfaßt eine konvexe Ersatzgleitfläche 12 mit einem exzentrisch zur Mittenachse 23 derselben angeordneten Verankerungszapfen 11, der konisch ausgebildet ist. Die Mittenachse des Verankerungszapfens 11 ist mit der Bezugsziffer 24 gekennzeichnet. Dementsprechend läßt sich die Ersatzgleitfläche 12 um die Mittenachse 24 des Verankerungszapfens 11 in Richtung des Doppelpfeiles 25 hin- und herdrehen, bis intraoperativ die geeignete oder optimale Drehposition der Ersatzgleitfläche 12 gefunden worden ist. Erst dann erfolgt die endgültige Verankerung bzw. Verkoppelung der Prothesenhälfte bzw. des dargestellten Gelenkeinsatzes.

Der in den Figuren 1 und 2 dargestellte Gelenkeinsatz ist mit der Bezugsziffer 10 gekennzeichnet.

In den Figuren 3 und 4 ist eine komplementär zu der proximalen Prothesenhälfte gemäß den Figuren 1 und 2 ausgebildete distale Prothesenhälfte 26 dargestellt. Diese umfaßt eine konkave Ersatzgleitfläche 16 mit einer Mittenachse 27, und einen konischen Verankerungszapfen 14 mit einer Mittenachse 28. Die beiden Mittenachsen 27, 28 sind ebenso wie die Mittenachsen 23, 24 der proximalen Prothesenhälfte versetzt zueinander. Dieser Versatz definiert eine vorbestimmte Exzentrizität "e".

Die Rückseiten der Gelenkeinsätze 10 und 26 bzw. die den Ersatzgleitflächen gegenüberliegenden Seiten sind mit den Bezugsziffern 13 bzw. 15 gekennzeichnet.

Vor endgültiger Verblockung des distalen Gelenkeinsatzes bzw. der distalen Prothesenhälfte 26 läßt sich diese um die Zapfen-Mittenachse 28 in Richtung des Doppelpfeiles 29 hin- und herverdrehen, bis eine optimale Position der zugeordneten Ersatzgleitfläche 16 erhalten ist. Dann erfolgt die endgültige Verblockung im Knochen oder einem der Prothesenhälfte zugeordneten Anschlußelement, welches im Knochen zementfrei oder durch Zementierung verankert ist.

Wie dies im einzelnen aussehen kann, wird nunmehr anhand der Figuren 5 und 6 näher beschrieben. Fig. 5 zeigt ein Beispiel für eine Totalprothese für ein Metatarsophalgeal- bzw. MTP-Gelenk in röntgenologischer Seitenansicht. Mit der Bezugsziffer 30 ist die sogenannte Phalanx und mit der Bezugsziffer 17 die sogenannte Metatarsale I gekennzeichnet. Das Großzehengelenk ist insgesamt mit der Bezugsziffer 31 angegeben. Am distalen Ende der Metatarsale 17 ist eine kugelkalottenartige Prothesenhälfte 10 mit entsprechender Ersatzgleitfläche 12 fixiert, die mit einer komplementären konkaven Gleitfläche 16 einer am proximalen Ende der Phalanx 30 implantierten Prothesenhälfte 26 entsprechend den Figuren 3 und 4 korrespondiert.

Die proximale Prothesenhälfte 10 entsprechend den Figuren 1 und 2 ist mit einem am distalen Ende der Metatarsale 17 implantierten Anschlußelement 18 verankert, wobei die Verankerung durch eine selbsthemmende Steckverbindung zwischen dem konischen Verankerungszapfen 11 und einer komplementär konischen Zapfenaufnahme bzw. -bohrung 19 erfolgt. Das Anschlußelement 18 ist innerhalb der Metatarsale zementfrei oder alternativ durch Zementierung verankert. Diesbezüglich handelt es sich um eine an sich bekannte Technik, auf die nicht näher eingegangen werden muß. Ergänzend dazu sei auch auf die bereits eingangs erwähnte EP 0 827 386 B1 verwiesen. Wesentlich ist das Zusammenspiel zwischen der zentrisch oder ebenfalls exzentrisch vorbereiteten Zapfenaufnahme 19 und dem relativ zur Ersatzgleitfläche 12 exzentrisch angeordneten Verankerungszapfen 11.

In gleicher Weise kann auch die komplementäre Prothesenhälfte 26 in der Phalanx verankert sein. Auch dort ist ein gesondertes Anschlußelement 20 im Knochen, nämlich in der Phalanx 30 implantiert. Mit diesem Anschlußelement läßt sich der distale Gelenkeinsatz gemäß den Figuren 3 und 4 bzw. die entsprechende Prothesenhälfte 26 verkoppeln, wobei auch hier wiederum eine Prothesenhälfte mit exzentrischem Verankerungszapfen 14 zum Einsatz kommen kann.

Es sei darauf hingewiesen, daß es auch denkbar ist, die Zapfen-Aufnahmebohrungen 19, 21 exzentrisch innerhalb der Anschlußelemente 18, 20 auszubilden, wobei die Exzentrizität vorzugsweise dieselbe ist wie zwischen Ersatzgleitfläche einerseits und Verankerungszapfen andererseits. Auch das Anschlußelement 20 weist also eine Zapfenaufnahme 21 entsprechend dem konisch ausgebildeten Verankerungszapfen 14 auf.

In Fig. 6 ist eine Halbprothese mit exzentrischem distalem Gelenkeinsatz bzw. Prothesenhälfte 26 dargestellt, und zwar in einer Art wie in Fig. 5. Dementsprechend ist also nur am proximalen Ende der Phalanx 30 eine Prothesenhälfte 26 mit konkaver Ersatzgleitfläche 16 implantiert. Die Prothesenhälfte 26 ist entsprechend den Figuren 3 und 4 ausgebildet und weist einen exzentrisch zur Ersatzgleitfläche 16 angeordneten Verankerungszapfen 14 auf. Dieser korrespondiert mit einer komplementären Zapfen-Aufnahmebohrung 21 eines vorab implantierten Anschlußelements 20.

Zur Fig. 5 sei noch erwähnt, daß es dort nicht unbedingt erforderlich ist, daß auch die Verankerungsmittel für den distalen Gelenkeinsatz 26 exzentrisch zur zugeordneten Ersatzgleitfläche ausgebildet sind.

In den Figuren 5 und 6 ist das sogenannte Sesambeinchen noch jeweils mit der Bezugsziffer 22 gekennzeichnet.

Die beschriebene Konstruktion läßt sich auch an anderen Zehengelenken oder Fingergelenken gestalten. Insofern soll die Anwendung der beschriebenen Erfindung nicht beschränkt sein.

Es sei auch noch darauf hingewiesen, daß beim Implantat für die Phalanx bzw. Grundphalanx 30 unterschiedliche Plateauhöhen möglich sind, wodurch man eine ausgewogene Kapselspannung wieder herstellen bzw. erzielen kann.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen:

- 10: proximaler Gelenkeinsatz bzw. proximale Prothesenhälfte
- 11: Verankerungszapfen
- 12: konvexe Ersatzgleitfläche
- 13: Rückseite
- 14: Verankerungszapfen
- 15: Rückseite
- 16: konkave Ersatzgleitfläche
- 17: Metatarsale
- 18: Anschlußelement
- 19: Zapfenaufnahme
- 20: Anschlußelement
- 21: Zapfenaufnahme
- 22: Sesambeinchen
- 23: Mittenachse
- 24: Mittenachse
- 25: Doppelpfeil
- 26: distaler Gelenkeinsatz bzw. distale Prothesenhälfte
- 27: Mittenachse
- 28: Mittenachse
- 29: Doppelpfeil
- 30: (Grund)phalanx
- 31: Großzehengelenk
- e: Exzentrizität

## Patentansprüche

1. Endoprothese für ein Metatarsophalangeal (MTP)-Gelenk, insbesondere Großzehengrundgelenk, Zehen- oder Fingergelenk, mit einer eine konkave (16) oder konvexe (12) Ersatzgleitfläche definierenden Prothesenhälfte (26; 10), wobei exzentrisch (e) zu den Ersatzgleitflächen (12; 16) der Prothesenhälften (26; 10) angeordnete Mittel (11, 19 bzw. 14, 21) zur Verankerung derselben im Knochen (17; 30) vorgesehen sind, die jeweils als Verankerungszapfen (14; 11) ausgebildet sind, dessen Mittenachse (28; 24) relativ zur Mittenachse (27; 23) der konkaven (16) bzw. konvexen (12) Ersatzgleitfläche versetzt ist,
**dadurch gekennzeichnet, daß**
sie modular aufgebaut ist und neben der Prothesenhälfte (26; 10) mit Ersatzgleitflächen (16; 12) ein im Knochen zementfrei oder durch Zementierung verankerbares Anschlußelement (20; 18) für die Prothesenhälfte aufweist.

2. Endoprothese nach Anspruch 1,
**dadurch gekennzeichnet, daß**
die Prothesenhälfte (26; 10) am Anschlußelement (20; 18) durch Schrauben oder über eine selbsthemmende Steckverbindung lösbar fixiert ist.

3. Endoprothese nach Anspruch 2,
**dadurch gekennzeichnet, daß**
die selbsthemmende Steckverbindung durch einen konischen Verankerungszapfen (14; 11) einerseits und eine komplementär konische Zapfenaufnahme (21; 19) andererseits gebildet ist, wobei die Mittenachse (28; 24) von Zapfen (14; 11) und gegebenenfalls auch Zapfenaufnahme (21; 19) relativ zur Mittenachse (27; 23) der konkaven (16) bzw. konvexen (12) Ersatzgleitfläche versetzt ist.

4. Endoprothese nach einem der Ansprüche 1 bis 3,
**dadurch gekennzeichnet, daß**
die Mittenachsen (27; 23) von Ersatzgleitflächen (16; 12) und Prothesenverankerung (14; 11) sich entweder parallel oder in einem vorbestimmten Winkel zueinander erstrecken.

5. Endoprothese nach einem der Ansprüche 1 bis 4,
**dadurch gekennzeichnet, daß**
bei einer Schraubfixierung der Prothesenhälfte (26; 10) am Anschlußelement (20; 18) die Prothesenhälfte oder ein Befestigungsteil derselben einen geradlinig oder bogenförmig verlaufenden langlochartigen Durchgang für eine Befestigungsschraube aufweist, wobei dieser Durchgang vorzugsweise außerhalb der orthopädisch wirksamen Ersatzgleitflächen (16; 12) liegt.

6. Endoprothese nach einem der Ansprüche 1 bis 4,
**dadurch gekennzeichnet, daß**
bei einer Schraubfixierung der Prothesenhälfte (26; 10) am Anschlußelement (20; 18) letzteres mehrere voneinander beabstandete Gewindebohrungen zur Aufnahme einer der Prothesenhälfte zugeordneten Befestigungsschraube aufweist derart, daß die Prothesenhälfte mit ihrer Ersatzgleitfläche (16; 12) entweder zentrisch oder bei Bedarf exzentrisch zur Mittenachse des Anschlußelements (20; 18) an diesem befestigbar ist.

7. Endoprothese nach Anspruch 5 oder 6,
**dadurch gekennzeichnet, daß**
der Durchgang für die Befestigungsschraube in der Prothesenhälfte (26; 10) etwas größer dimensioniert ist als der Durchmesser der Befestigungsschraube bzw. des Schaftes derselben, so daß die Prothesenhälfte (26; 10) vor endgültiger Verblockung durch die Befestigungsschraube nach allen Seiten hin (in eine zentrische oder exzentrische Lage) verschiebbar ist.

## Claims

1. Endoprosthesis for a metatarsophalangeal joint (MTP), in particular, a basal joint of a big-toe, a toe joint or a finger joint, with a prosthesis half (26; 10) defining a concave (16) or convex (12) prosthetic gliding surface, wherein means (11, 19 or respectively 14, 21) for anchoring the same within the bone (17; 30) disposed eccentrically (e) to the prosthetic gliding surfaces (12; 16) of the prosthesis halves (26; 10) are provided, which means are formed respectively as anchoring pins (14; 11), of which the central axis (28; 24) is offset relative to the central axis (27; 23) of the concave (16) or respectively convex (12) prosthetic gliding surface,
**characterised in that**
it is structured in a modular manner and, in addition to the prosthesis half (26; 10) with the prosthetic gliding surface (16; 12), provides a connecting element (20; 18) for the prosthesis half, which can be anchored within the bone either in a cementless manner or by cementing.

2. Endoprosthesis according to claim 1,
**characterised in that**
the prosthesis half (26; 10) is fastened to the connecting element (20; 18) in a detachable manner by a screw connection or via a self-locking plug connection.

3. Endoprosthesis according to claim 2,
**characterised in that**
the self-locking plug connection is formed on one side by a conical anchoring pin (14; 11) with a complementary conical-pin receiver (21; 19) on the other side, wherein the central axis (28; 24) of the pin (14; 11), and optionally also of the pin receiver (21; 19), is offset relative to the central axis (27; 23) of the concave (16) or convex (12) prosthetic gliding surface.

4. Endoprosthesis according to any one of claims 1 to 3,
**characterised in that**
the central axes (27; 23) of the prosthetic gliding surfaces (16; 12) and of the prosthesis-anchoring pin (14; 11) extend either parallel or at a predetermined angle relative to one another.

5. Endoprosthesis according to any one of claims 1 to 4,
**characterised in that,**
in the case of a screw fastening of the prosthesis half (26; 10) to the connecting element (20; 18), the prosthesis half or a fastening component thereof provides a slot-like passage extending in a straight-line or in a curve for a fastening screw, wherein this passage is preferably disposed outside the orthopaedically-active prosthetic gliding surfaces (16; 12).

6. Endoprosthesis according to any one of claims 1 to 4,
**characterised in that,**
in the case of a screw fastening of the prosthesis half (26; 10) to the connecting element (20; 18), the latter provides several threaded boreholes disposed at a spacing distance from one another to receive one of the fastening screws associated with the prosthesis half, in such a manner that the prosthesis half with its prosthetic gliding surface (16; 12) can be fastened to the latter either concentrically, or if required, eccentrically, relative to the central axis of the connecting element (20; 18).

7. Endoprosthesis according to claim 5 or 6,
**characterised in that**
the passage for the fastening screw in the prosthesis half (26; 10) is dimensioned somewhat larger than the diameter of the fastening screw or respectively of its shaft, so that the prosthesis half (26; 10) can be displaced towards all sides (into a concentric or an eccentric position) before the final locking by the fastening screw.

## Revendications

1. Endoprothèse pour articulation métatarsophalangienne (MTP), notamment l'articulation basale du gros orteil, l'articulation de l'orteil ou du doigt, avec une moitié de prothèse (26 ; 10) définissant une surface de glissement de substitution concave (16) ou convexe (12), dans laquelle il est prévu des moyens d'ancrage (11, 19 ou 14, 21) de la moitié de prothèse dans l'os (17 ; 30), lesquels moyens d'ancrage sont agencés de manière excentrée (e) par rapport aux surfaces de glissement de substitution (12 ; 16) des moitiés de prothèse (26 ; 10) et sont respectivement conçus sous la forme de tétons d'ancrage (14 ; 11) dont l'axe médian (28 ; 24) est décalé par rapport à l'axe médian (27 ; 23) de la surface de glissement de substitution concave (16) ou convexe (12),
**caractérisée en ce que**
ladite endoprothèse est de conception modulaire et comporte, outre la moitié de prothèse (26 ; 10) présentant les surfaces de glissement de substitution (16 ; 12), un élément de raccordement de la moitié de prothèse (20 ; 18) apte à être ancré dans l'os avec ou sans ciment.

2. Endoprothèse selon la revendication 1,
**caractérisée en ce que**
la moitié de prothèse (26 ; 10) est fixée de manière amovible à l'élément de raccordement (20 ; 18) par des vis ou un système d'enfichage autobloquant.

3. Endoprothèse selon la revendication 2,
**caractérisée en ce que**
le système d'enfichage autobloquant est formé, d'une part, par un téton d'ancrage conique (14 ; 11) et, d'autre part, par un logement de téton (21 ; 19) de forme complémentaire conique, l'axe médian (28 ; 24) du téton (14 ; 11) et éventuellement également du logement de téton (21 ; 19) étant décalé par rapport à l'axe médian (27 ; 23) de la surface de glissement de substitution concave (16) ou convexe (12).

4. Endoprothèse selon l'une quelconque des revendications 1 à 3,
**caractérisée en ce que**
les axes médians (27 ; 23) des surfaces de glissement de substitution (16 ; 12) et de l'ancrage de prothèse (14 ; 11) sont parallèles ou forment entre eux un angle prédéfini.

5. Endoprothèse selon l'une quelconque des revendications 1 à 4,
**caractérisée en ce que**
dans le cas d'une fixation par vissage de la moitié de prothèse (26 ; 10) à l'élément de raccordement (20 ; 18), la moitié de prothèse ou un élément de fixation de celle-ci présente un trou oblong droit ou en arc de cercle pour le passage d'une vis de fixation, ce passage étant de préférence situé en dehors des surfaces de glissement de substitution (16 ; 12) utiles sur le plan orthopédique.

6. Endoprothèse selon l'une quelconque des revendications 1 à 4,
**caractérisée en ce que**
dans le cas d'une fixation par vissage de la moitié de prothèse (26 ; 10)) à l'élément de raccordement (20 ; 18), celui-ci présente une pluralité de trous taraudés distants les uns des autres, destinés à recevoir une vis de fixation associée à la moitié de prothèse de manière à ce que la moitié de prothèse puisse être fixée audit moyen de raccordement avec la face de glissement de substitution (16 ; 12) soit centrée, soit excentrée, si nécessaire, par rapport à l'axe médian de l'élément de raccordement (20 ; 18).

7. Endoprothèse selon la revendication 5 ou la revendication 6,
**caractérisée en ce que**
le passage pour la vis de fixation ménagé dans la moitié de prothèse (26 ; 10) est dimensionné légèrement plus grand que le diamètre ou la partie lisse de la vis de fixation de manière à ce que la moitié de prothèse (26 ; 10) puisse être déplacée dans toutes les directions avant le blocage définitif par la vis de fixation (dans une position centrée ou excentrée).
